(19) Europäisches Patentamt
European Patent Office
Office européen des brevets

(11) Veröffentlichungsnummer: **0 066 303**
**B1**

(12) # EUROPÄISCHE PATENTSCHRIFT

(45) Veröffentlichungstag der Patentschrift: **19.06.85**

(51) Int. Cl.[4]: **C 07 D 243/16,** A 61 K 31/55

(21) Anmeldenummer: **82107036.4**

(22) Anmeldetag: **10.12.80**

(60) Veröffentlichungsnummer der früheren Anmeldung nach Art. 76 EPÜ:

(54) **2-Azidomethyl-1,4-benzodiazepine und deren Salze sowie Verfahren zu ihrer Herstellung und diese Verbindungen enthaltende Arzneimittel.**

(30) Priorität: **24.12.79 DE 2952279**

(43) Veröffentlichungstag der Anmeldung: **08.12.82 Patentblatt 82/49**

(45) Bekanntmachung des Hinweises auf die Patenterteilung: **19.06.85 Patentblatt 85/25**

(84) Benannte Vertragsstaaten: **AT BE CH DE FR GB IT LI LU NL SE**

(56) Entgegenhaltungen:
**DE-A-2 314 993**
**DE-A-2 353 160**
**DE-A-2 436 147**

(73) Patentinhaber: **Kali-Chemie Pharma GmbH, Hans-Böckler-Allee 20 Postfach 220, D-3000 Hannover 1 (DE)**

(72) Erfinder: **Zeugner, Horst, Dipl.-Chem., Dr.rer.nat., Havelweg 10, D-3000 Hannover 73 (DE)**
Erfinder: **Römer, Dietmar, Dr., Pharm., Lettenweg 111, CH-4123 Allschwil (CH)**
Erfinder: **Liepmann, Hans, Dipl.-Chem., Dr.rer.nat., Auf dem Emmerberg 17, D-3000 Hannover 1 (DE)**
Erfinder: **Milkowski, Wolfgang, Dipl.-Chem., Dr.rer.nat., Fasanenweg 13, D-3167 Burgdorf (DE)**

(74) Vertreter: **Lauer, Dieter, Dr., c/o Kali-Chemie Aktiengesellschaft Postfach 220, D-3000 Hannover 1 (DE)**

**EP 0 066 303 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Patentanmeldung ist eine Ausscheidungsanmeldung aus der europäischen Patentanmeldung 80 107 779.3, welche neue 2-Acylaminoethyl-1,4-benzodiazepine betrifft.

Die vorliegende Erfindung betrifft neue 2-Azidomethyl-1,4-benzoediazepine, deren Salze sowie Verfahren zu ihrer Herstellung und diese Verbindungen enthaltende pharmazeutische Zubereitungen.

In der DE-OS 2 353 187 sind u. a. 2-Acylaminomethyl-1,4-benzoediazepine beschrieben, in welchen der Acylrest ein niedermolekulares Alkanoyl ist. Diese Substanzen besitzen vor allem eine antikonvulsive Wirkung.

Ziel der Erfindung ist es, neue Zwischenprodukte zur Herstellung neuer 2-Acylaminomethyl-1,4-benzodiazepine mit neuartigem Wirkungsprofil zu entwickeln.

Überraschenderweise wurde dabei gefunden, daß die erfindungsgemäßen 2-Azidomethyl-1,4-benzodiazepine wertvolle Zwischenprodukte darstellen, welche weiter umgesetzt werden können zu neuen 2-Acylaminomethyl-1,4-benzodiazepinen, welche neben psychopharmakologischen, diuretischen und antiarrhythmischen Wirkungen vor allem ausgeprägte analgetische Eigenschaften bei geringer Toxizität aufweisen. Diese neuen 2-Acylaminomethyl-1,4-benzodiazepine, ihre pharmakologischen Eigenschaften und ihre Herstellung aus den erfindungsgemäßen 2-Azidomethyl-1,4-benzodiazepinen sind in der europäischen Patentanmeldung 80 107 779.3 ausführlich beschrieben und beansprucht.

Außerdem weisen die erfindungsgemäßen 2-Azidomethyl-1,4-benzodiazepine selbst wertvolle pharmakologische Eigenschaften auf.

Gegenstand der Erfindung sind 2-Azidomethyl-1H-2,3-dihydro-1,4-benzodiazepine der allgemeinen Formel I

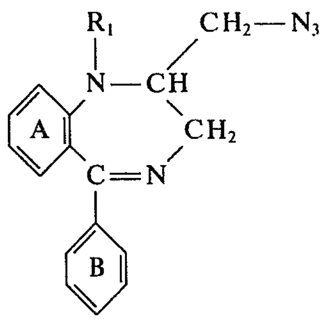

(I)

worin $R_1$ ist ein Wasserstoffatom oder eine Alkylgruppe mit 1—4 Kohlenstoffatomen, A und B sind unabhängig voneinander unsubstituiert oder substituiert mit 1 bis 3 Substituenten ausgewählt aus der Gruppe bestehend aus Halogen, Alkylthio mit 1—4 Kohlenstoffatomen, Alkoxy mit 1—4 Kohlenstoffatomen, Alkyl mit 1—4 Kohlenstoffatomen, Hydroxy, Nitro und Trifluormethyl, oder A und B sind Phenylreste, in welchen zwei benachbarte C-Atome durch eine Methylendioxy- oder Äthylendioxygruppe verbunden sind, sowie deren optische Isomeren und die Säureadditionssalze der Verbindungen der allgemeinen Formel I.

Als Alkylgruppen $R_1$ kommen niedermolekulare Gruppen mit bis zu 4 Kohlenstoffatomen in Frage, die gerade oder verzweigt sind, z. B. Methyl, Äthyl, Propyl, Isopropyl, n-Butyl, 1-Methylpropyl, 2-Methylpropyl oder tert.-Butyl.

Die Alkylreste in den mit Alkyl, Alkylthio oder Alkoxy substituierten Phenylringen A und/oder B haben ebenfalls die obige Bedeutung. Insbesondere bei Di- und Trisubstitution an den Phenylringen sind der Methyl- und der Äthylrest bevorzugt. Als Halogenatome kommen Fluor, Chlor oder Brom in Frage. Für Alkylthio, Nitro und Trifluormethyl ist die Monosubstitution bevorzugt, für Halogen und/oder Alkyl bzw. und/oder Alkoxy oder Hydroxy Mono- und Disubstitution. Für niedermolekulares Alkoxy, insbesondere Methoxy, ist auch die Trisubstitution begünstigt.

Gegenstand der Erfindung ist auch ein Verfahren zur Herstellung der Verbindungen der allgemeinen Formel I. Die neuen Verbindungen werden dadurch erhalten, daß man in an sich bekannter Weise Verbindungen der allgemeinen Formeln II oder III oder deren Gemische

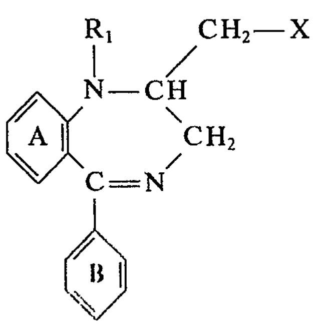

(II)

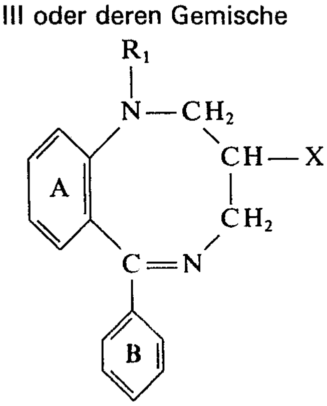

(III)

worin A und B und $R_1$ die oben angegebenen Bedeutungen haben und X Halogen, vorzugsweise Chlor, ist mit einem Alkalimetallazid, vorzugsweise Natrium- oder Kaliumazid, in einem inerten Lösungsmittel bei Temperaturen von −30 bis 150°C umsetzt. Die erhaltenen 2-Azidomethyl-1,4-benzodiazepine der allgemeinen Formel I können in Form ihrer Basen oder Säureadditionssalze isoliert werden, sie können aber auch, gegebenenfalls nach Entfernung des Lösungsmittels, ohne Abtrennung aus dem Reaktionsgemisch, weiter umgesetzt werden.

Verbindungen der Formel I können in die optisch aktiven Formen aufgetrennt werden.

Die Auftrennung in die optisch aktiven Verbindungen kann aber auch in einer geeigneten Vorstufe erfolgen.

Die Herstellung der Ausgangssubstanzen kann in an sich bekannter Weise nach Verfahren erfolgen, die in der DE-OS 2 221 558 beschrieben sind. Ausgangsprodukte für die Verbindungen der Formel II und III sind danach 1-Acyl-2-hydroxy-1,3-diaminopropane, deren Herstellung in den Deutschen Offenlegungsschriften 2 221 558, 2 314 993, 2 720 915 und 2 720 968 beschrieben ist.

Danach kann man 2-Hydroxy-1,3-diaminopropane der allgemeinen Formel V,

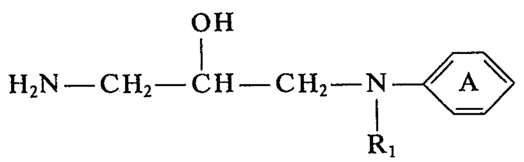

(V)

in der $R_1$ und A die oben angegebenen Bedeutungen haben, mit einem gegebenenfalls substituierten Benzoylchlorid zu Verbindungen der allgemeinen Formel VI,

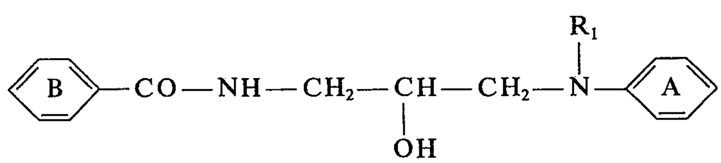

(VI)

in der A und B und $R_1$ die oben angegebenen Bedeutungen haben, umsetzen.

Die Ausgangsverbindungen der allgemeinen Formel V können auf die von M. Chadwick et al. in J. Med. Chem. 9, S. 874 (1966) beschriebene Weise hergestellt werden.

Die Verbindungen der allgemeinen Formel VI, in denen $R_1$ die Bedeutung Wasserstoff hat, können auf dieser Stufe durch nachträgliche Alkylierung in an sich bekannter Weise in die entsprechenden N-Alkylverbindungen übergeführt werden. Dies geschieht beispielsweise nach den aus der Literatur bekannten Verfahren der reduktiven Carbonyl-Aminierung wie der Leuckart-Wallach- bzw. Eschweiler-Clarke-Reaktion (s. H. Krauch, W. Kunz, Reaktionen der Organischen Chemie [1976], S. 126 und 131) oder durch Alkylierung mit Dialkylsulfaten (s. Houben-Weyl XI/1 [1957], S. 207 ff).

Die auf die vorstehende Weise erhaltenden Verbindungen der allgemeinen Formel VI können nun in an sich bekannter Weise, wie bereits in den Deutschen Offenlegungsschriften 2 221 558, 2 314 993 und 2 520 937 beschrieben, durch Umsetzung mit Phosphoroxidhalogeniden, vorzugsweise Phosphoroxidchlorid, cyclisiert werden. Man behandelt dazu zweckmäßigerweise Verbindungen der allgemeinen Formel VI oder deren Säureadditionssalze, wie in DE-OS 2 520 937 beschrieben, mit dem Cyclisierungsmittel bei einer Temperatur zwischen 100 und 150°C und isoliert anschließend das Gemisch der beiden isomeren Verbindungen mit den allgemeinen Formeln II und III.

Die beiden isomeren Verbindungen II und III liegen im Reaktionsgemisch in wechselnden Mengenverhältnissen von II zu III vor in Abhängigkeit von der Art der Substituenten an den Phenylringen A und B, sowie von deren Stellung. Dies ist jedoch für die folgende Umsetzung dieses Gemisches ohne Belang, da beide Isomere in einheitlicher Reaktion zu Verbindungen der allgemeinen Formel I, reagieren. Eine langwierige Trennung oder Analyse des Isomerengemisches ist daher nicht nötig.

So kann man das wie oben beschrieben erhaltene Isomerengemisch von Verbindungen der allgemeinen Formel II und III nach grober Abtrennung von Nebenprodukten, jedoch ohne Trennung in die Einzelkomponenten mit Alkalimetallazid umsetzen.

Geeignete Lösungsmittel für die Herstellung der Azide der Formel I sind beispielsweise Methylenchlorid, Chloroform, Tetrahydrofuran, Dioxan, Dimethylformamid, Dimethylsulfoxid, Hexamethylphosphortriamid, Methanol, Äthanol, t-Butanol, Aceton oder Methylisobutylketon.

Die Verbindungen der allgemeinen Formel I sind neue Substanzen und wertvolle Zwischenprodukte für die Herstellung von neuen 2-Acylaminomethyl-1,4-benzodiazepinen, welche in der Europäischen Patentanmeldung 80 107 779,3 beschrieben sind. Darüber hinaus weisen sie auch selbst wertvolle pharmakologische Wirkungen auf. Psychopharmakologische Wirkungen der erfindungsgemäßen Verbindungen wurden in Standard-Screening-Tests der PANLABS Inc. in Mäusen nachgewiesen, desgleichen bronchodilatorische Wirkungen an der durchströmten Meerschweinchenlunge und antiarrhythmische Effekte nach Chloroform induzierter Arrhythmie am Mäuseherzen.

Die pharmakologischen Teste ergaben im Dosisbereich von 0,5—100 mg/kg gute Ergebnisse und die Verbindungen eignen sich daher therapeutisch als Sedativa, Broncholytika und Antiarrhythmika.

Durch reduktive Spaltung der 2-Azidomethyl-1,4-benzodiazepine in an sich bekannter Weise mit Hydrazinhydrat/Raney-Nickel unter basischer Katalyse oder mit Propandithiol erhält man die entsprechenden 2-Aminomethyl-1,4-benzodiazepine. Die Reduktion mit Hydrazin erfolgt zweckmäßigerweise in Alkoholen wie Methanol oder Äthanol unter Zusatz von tertiären Aminen wie Triäthylamin bei Raumtemperatur. Bei der Reduktion mit Propandithiol dienen Methanol, Äthanol, Dimethylformamid oder Pyridin/Wasser als Lösungsmittel.

Auf an sich bekannte Weise können die 2-Aminomethylverbindungen weiter zu 2-Acylaminomethyl-1,4-benzodiazepinen acyliert werden.

Ausdrücklich soll an dieser Stelle betont werden, daß für die oben angegebenen Reaktionen zur Herstellung der Verbindungen der allgemeinen Formel I zwar zweckmäßigerweise das Isomerengemisch der Verbindungen II und III verwendet werden kann, doch ist es für den Fachmann offensichtlich, daß man das Isomerengemisch auch in die Einzelkomponenten trennen und diese dann separat in der angegebenen Reaktion zu Verbindungen der allgemeinen Formel I umsetzen kann.

Eine nachträgliche Substitution des 1,4-Benzodiazepinsystems ist im Phenylring A in an sich bekannter Weise durch Halogen oder die Nitrogruppe möglich, wie in der DE-OS 2 221 558 bereits beschrieben. Zweckmäßigerweise bieten sich als geeignete Produkte für eine solche Substitution Verbindungen der allgemeinen Formel I und II an. Als Halogenierungsmittel können beispielsweise N-Chlorsuccinimid oder N-Bromsuccinimid dienen. Zur Einführung der Nitrogruppe können die üblichen Nitrierungsreagenzien herangezogen werden, beispielsweise $KNO_3$ in $H_2SO_4$ oder Kupfer-II-nitrat-trihydrat in Acetanhydrid.

Die nachfolgenden Beispiele erläutern die Herstellung der neuen Verbindungen der allgemeinen Formel I, sie sollen jedoch den Umfang der Erfindung in keiner Weise beschränken.

Die Strukturen der neuen Verbindungen wurden durch spektroskopische Untersuchungen, insbesondere durch eine genaue Analyse der NMR-Spektren gesichert. In den Tabellen werden, wenn keine weiteren Angaben vorliegen, die Schmelzpunkte der Monohydrochloride angegeben. Evtl. Vorliegen von eingeschlossenen Mengen an Wasser, Aceton, Äthanol oder dgl. werden angeführt.

## Beispiel 1

### 1-Methyl-2-azidomethyl-5-phenyl-1H-2,3-dihydro-1,4-benzodiazepin

a) 202 g $N_1$-Benzoyl-$N_2$-methyl-$N_2$-phenyl-2-hydroxy-1,3-diaminopropan wurden in 1000 ml Phosphoroxidchlorid 2,5 Stunden unter Rückfluß gekocht. Dann wurde das überschüssige Phosphoroxidchlorid abdestilliert und der Rückstand in Chloroform (1000 ml) aufgenommen. Die Chloroformlösung wurde mit 1000 ml Eis/Wasser durchgerührt, die organische Phase wurde abgetrennt und 5- bis 6mal mit 200 ml Wasser gewaschen. Anschließend wurde die organische Phase mit 1200 ml Natronlauge (20%) geschüttelt und darauf mit Wasser gewaschen. Die Chloroformphase wurde dann durch Zusatz von Natriumsulfat und $\gamma$-Tonerde (Aluminiumoxid »Giulini«) getrocknet und entfärbt. Nach dem Filtrieren wurde die Lösung eingedampft.

b) 68,4 g des nach Beispiel 1a erhaltenen Cyclisierungsgemisches aus 1-Methyl-2-chlormethyl-5-phenyl-1H-2,3-dihydro-1,4-benzodiazepin und 1-Methyl-3-chlor-6-phenyl-1,2,3,4-tetrahydrobenzodiazocin wurden mit 47,2 g Natriumazid in 450 ml Dimethylformamid 4 Stunden auf 100°C erwärmt. Danach wurde das Dimethylformamid im Vakuum abdestilliert und der Rückstand zwischen 300 ml Toluol und 200 ml Wasser verteilt. Die organische Phase wurde abgetrennt, mit Kochsalzlösung (10%) gewaschen, über Natriumsulfat getrocknet und filtriert. Dann wurde das Lösungsmittel abdestilliert. Es werden 56,1 g rohes 1-Methyl-2-azidomethyl-5-phenyl-1H-2,3-dihydro-1,4-benzodiazepin als Rückstand erhalten. Der Rückstand wurde in Äther gelöst und mit einer gesättigten Lösung von Chlorwasserstoff in Äther versetzt. Die ausfallenden Kristalle wurden abfiltriert und aus Aceton/Isopropanol umkristallisiert.

Es wurden 36,7 g 1-Methyl-2-azidomethyl-5-phenyl-1H-2,3-dihydro-1,4-benzodiazepinhydrochlorid mit Fp: 181—183°C erhalten.

Nach dem in Beispiel 1 beschriebenen Verfahren können durch Umsetzung von entsprechenden Verbindungen der Formel II und/oder III mit Natriumazid die folgenden substituierten 2-Azidomethyl-5-phenyl-1H-2,3-dihydro-1,4-benzodiazepine der allgemeinen Formel I hergestellt werden, in welchen die Substituenten in den Ringen A und B und $R_1$ folgende Bedeutung haben:

| A | B | $R_1$ | Schmelzpunkt, °C Hydrochlorid |
|---|---|---|---|
| 7-$CF_3$ | H | $CH_3$ | 175–177 |
| 7-F | 3,4,5-$OCH_3$ | $CH_3$ | 123–126 Base |
| 7-$CH_3$ | 3,4,5-$OCH_3$ | $CH_3$ | 187–188 |
| 8-$OCH_3$ | H | $CH_3$ | 193–194 |
| 7-$OCH_3$ | H | $CH_3$ | 203–205 |
| H | H | $CH_3$ | 187–190 |
| 7-Cl | 2-Cl | $CH_3$ | 170–173 |
| 7-F | H | $CH_3$ | 145–149 0,25 $H_2O$ |
| 7-Cl | H | $CH_3$ | 147–151,5 |
| H | 2-F | $C_2H_5$ | Öl*) |
| 7-Br | H | $CH_3$ | Öl*) |
| H | 2-Br | $CH_3$ | Öl*) |
| 7-$NO_2$ | H | $CH_3$ | Öl*) |
| 8-F | H | H | Öl*) |

*) IR-Spektrum: 2120 $cm^{-1}$ (Azid/Base)
Perkin-Elmer IR-Spektrophotometer 157 G

**Patentansprüche für die Vertragsstaaten: BE, CH, DE, FR, GB, IT, LI, LU, NL, SE**

1. 2-Azidomethyl-5-phenyl-1H-2,3-dihydro-1,4-benzodiazepine der allgemeinen Formel I

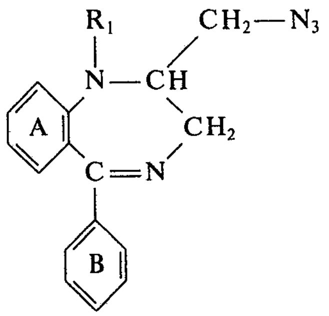

(I)

worin

$R_1$ ist ein Wasserstoffatom oder eine Alkylgruppe mit 1–4 Kohlenstoffatomen,
A und B sind unabhängig voneinander unsubstituiert oder substituiert mit 1 bis 3 Substituenten, ausgewählt aus der Gruppe bestehend aus Halogen, Alkylthio mit 1–4 Kohlenstoffatomen, Alkoxy mit 1–4 Kohlenstoffatomen, Alkyl mit 1–4 Kohlenstoffatomen, Hydroxy, Nitro und Trifluormethyl, oder
A und B sind Phenylreste, in welchen zwei benachbarte C-Atome durch eine Methylendioxy- oder Äthylendioxygruppe verbunden sind,

sowie deren Säureadditionssalze.

2. 2-Azidomethyl-5-phenyl-1H-2,3-dihydro-1,4-benzodiazepine der allgemeinen Formel I gemäß Anspruch 1, worin

Ring A unsubstituiert ist oder substituiert ist mit 7-Fluor, 8-Fluor, 7-Chlor, 7-Brom, 7-Methoxy, 8-Methoxy, 7-Methyl, 7-Trifluormethyl oder 7-Nitro,
Ring B Phenyl, 2-Fluorphenyl, 2-Chlorphenyl, 2-Bromphenyl oder 3,4,5-Trimethoxyphenyl bedeutet und
$R_1$ Wasserstoff, Methyl oder Äthyl bedeutet.

3. Verfahren zur Herstellung von 2-Azidomethyl-5-phenyl-1H-2,3-dihydro-1,4-benzodiazepinen der allgemeinen Formel I

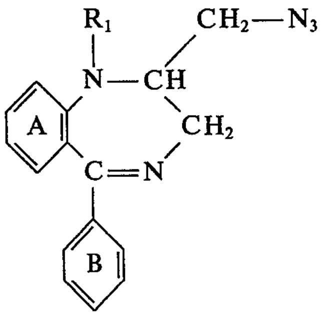

(I)

worin $R_1$ und die Phenylreste A und B die in Anspruch 1 angegebene Bedeutung besitzen, sowie deren Säureadditionssalze, dadurch gekennzeichnet, daß man Verbindungen der allgemeinen Formeln II und/oder III,

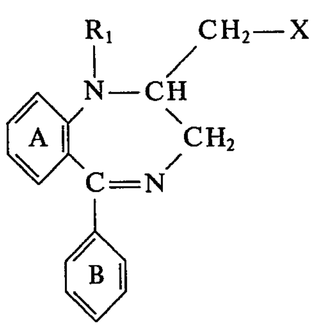

(II)

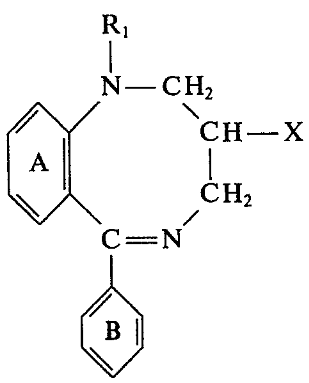

(III)

worin X Halogen bedeutet und $R_1$, A und B die obige Bedeutung haben, mit einem Alkalimetallazid, in einem inerten Lösungsmittel bei Temperaturen von −30 bis 105°C umsetzt und die Verbindungen der allgemeinen Formel I in Form ihrer Base oder ihre Säureadditionssalze isoliert.

4. Verfahren nach Anspruch 3, dadurch gekennzeichnet, daß man als Alkalimetallazid Natrium- oder Kaliumazid verwendet.

5. Arzneimittel, enthaltend eine oder mehrere Verbindungen gemäß Anspruch 1 und übliche pharmazeutische Hilfs- und Trägerstoffe.

**Patentansprüche für den Vertragsstaat: AT**

1. Verfahren zur Herstellung von 2-Azidomethyl-5-phenyl-1H-2,3-dihydro-1,4-benzodiazepinen der allgemeinen Formel I

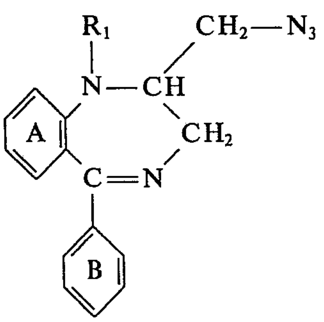

(I)

worin

R$_1$ ist ein Wasserstoffatom oder eine Alkylgruppe mit 1—4 Kohlenstoffatomen,
A und B sind unabhängig voneinander unsubstituiert oder substituiert mit 1 bis 3 Substituenten, ausgewählt aus der Gruppe bestehend aus Halogen, Alkylthio mit 1—4 Kohlenstoffatomen, Alkoxy mit 1—4 Kohlenstoffatomen, Alkyl mit 1—4 Kohlenstoffatomen, Hydroxy, Nitro und Trifluormethyl oder
A und B sind Phenylreste, in welchen zwei benachbarte C-Atome durch eine Methylendioxy- oder Äthylendioxygruppe verbunden sind,

sowie deren Säureadditionssalze, dadurch gekennzeichnet, daß man Verbindungen der allgemeinen Formeln II und/oder III,

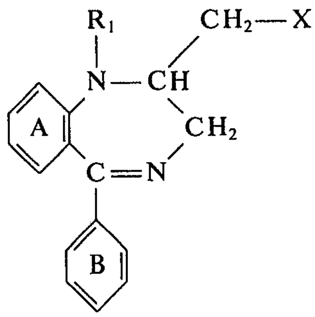
(II)

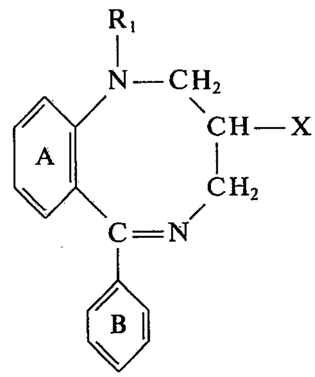
(III)

worin X Halogen bedeutet und R$_1$, A und B die obige Bedeutung haben, mit einem Alkalimetallazid, in einem inerten Lösungsmittel bei Temperaturen von −30 bis 105°C umsetzt und die Verbindungen der allgemeinen Formel I in Form ihrer Base oder ihre Säureadditionssalze isoliert.

2. Verfahren nach Anspruch 1, dadurch gekennzeichnet, daß man 2-Azidomethyl-5-phenyl-1H-2,3-dihydro-1,4-benzodiazepine der allgemeinen Formel I herstellt, worin

Ring A unsubstituiert ist oder substituiert ist mit 7-Fluor, 8-Fluor, 7-Chlor, 7-Brom, 7-Methoxy, 8-Methoxy, 7-Methyl, 7-Trifluormethyl oder 7-Nitro,
Ring B Phenyl, 2-Fluorphenyl, 2-Chlorphenyl, 2-Bromphenyl oder 3,4,5-Trimethoxyphenyl bedeutet und
R$_1$ Wasserstoff, Methyl oder Äthyl bedeutet.

3. Verfahren nach Anspruch 1, dadurch gekennzeichnet, daß man als Alkalimetallazid Natrium- oder Kaliumazid verwendet.

**Claims for the Contracting States: BE, CH, DE, FR, GB, IT, LI, LU, NL, SE**

1. 2-Azidomethyl-5-phenyl-1H-2,3-dihydro-1,4-benzodiazepines of the general formula I

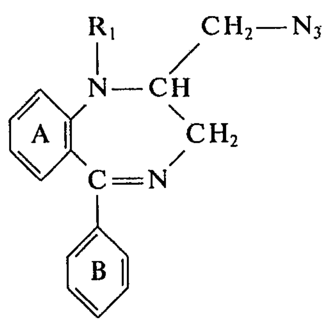
(I)

in which

R$_1$ is a hydrogen atom or an alkyl group with 1 to 4 carbon atoms,
A and B independently of each other are unsubstituted or substituted with 1 to 3 substituents, selected from the group consisting of halogen, alkylthio with 1 to 4 carbon atoms, alkoxy with 1 to 4 carbon atoms, alkyl with 1 to 4 carbon atoms, hydroxy, nitro and trifluoromethyl, or
A and B are phenyl radicals, in which two adjacent C-atoms are connected by a methylenedioxy or ethylenedioxy group,

and acid addition salts thereof.

2. 2-Azidomethyl-5-phenyl-1H-2,3-dihydro-1,4-benzodiazepines of the general formula I according to Claim 1, in which

Ring A is unsubtituted or is substituted with 7-fluoro, 8-fluoro, 7-chloro, 7-bromo, 7-methoxy, 8-methoxy, 7-methyl, 7-trifluoromethyl or 7-nitro,

Ring B is phenyl, 2-fluorophenyl, 2-chlorophenyl, 2-bromophenyl or 3,4,5-trimethoxyphenyl and

$R_1$ is hydrogen, methyl or ethyl.

3. Process for the preparation of 2-azidomethyl-5-phenyl-1H-2,3-dihydro-1,4-benzodiazepines of the general formuala I

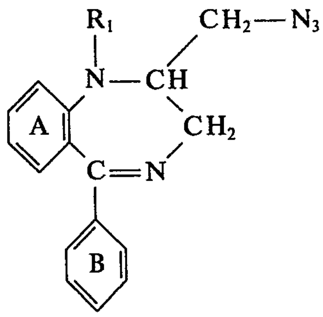

(I)

in which $R_1$ and the phenyl radicals A and B have the meaning given in Claim 1, and acid addition salts thereof, characterized in that compounds of the general formulae II and/or III

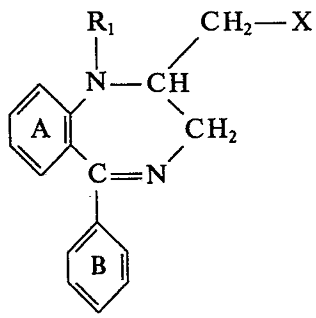

(II)

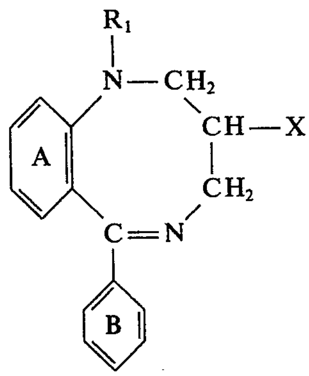

(III)

in which X signifies halogen and $R_1$, A and B have the above meaning, are reacted with an alkali metal azide, in an inert solvent at temperatures from −30 to 105°C and the compounds of the general formula I are isolated in the form of the base or their acid addition salts.

4. Process according to Claim 3, characterized in that the alkali metal azide is sodium or potassium azide.

5. Medicaments, containing one or more compounds according to Claim 1 and conventional pharmaceutical auxiliary and carrier substances.

**Claims for the Contracting State: AT**

1. Process for the preparation of 2-azidomethyl-5-phenyl-1H-2,3-dihydro-1,4-benzodiazepines of the general formula I

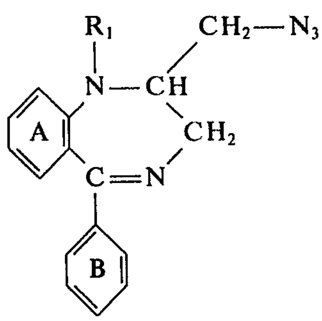

(I)

in which

R₁ is a hydrogen atom or an alkyl group with 1 to 4 carbon atoms,

A and B independently of each other are unsubstituted or substituted with 1 to 3 substituents, selected from the group consisting of halogen, alkylthio with 1 to 4 carbon atoms, alkoxy with 1 to 4 carbon atoms, alkyl with 1 to 4 carbon atoms, hydroxy, nitro and trifluoromethyl, or

A and B are phenyl radicals, in which two adjacent C-atoms are connected by a methylenedioxy or ethylenedioxy group,

and acid addition salts thereof characterized in that compounds of the general formulae II and/or III

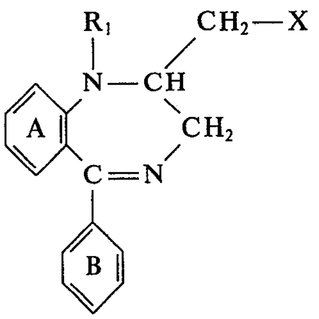

(II)

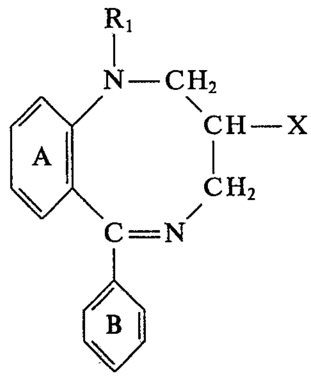

(III)

in which X is halogen and $R_1$, A and B have the above meaning, are reacted with an alkali metal azide, in an inert solvent at temperatures from −30 to 105°C and the compounds of the general formula I are isolated in the form of the base or their acid addition salts.

2. Process according to Claim 1, characterized in that 2-azidomethyl-5-phenyl-1H-2,3-dihydro-1,4-benzodiazepines of the general formula I according to Claim 1, in which

Ring A is unsubstituted or is substituted with 7-fluoro, 8-fluoro, 7-cloro, 7-bromo, 7-methoxy, 8-methoxy, 7-methyl, 7-trifluoromethyl or 7-nitro,

Ring B is phenyl, 2-fluorophenyl, 2-chlorophenyl, 2-bromophenyl or 3,4,5-trimethoxyphenyl and

$R_1$ is hydrogen, methyl or ethyl.

3. Process according to Claim 1, characterized in that the alkali metal azide is sodium or potassium azide.

**Revendications pour les etats contractants: BE, CH, DE, FR, GB, IT, LI, LU, NL, SE**

1. Azidométhyl-2 phényl-5 (1H) dihydro-2,3 benzodiazépines-1,4 de formule générale I

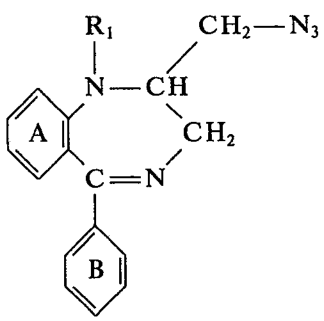

(I)

dans laquelle

$R_1$ est un atome d'hydrogène ou un groupe alkyle à 1 à 4 atomes de carbone,

A et B sont, indépendamment l'un de l'autre, non substitués ou substitués par 1 à 3 substituants choisis parmi un atome d'halogène, un groupe alkylthio à 1 à 4 atomes de carbone alcoxy à 1 à 4 atomes de carbone, alkyle à 1 à 4 atomes de carbone, hydroxy, nitro et trifluorométhyle ou

A et B sont des radicaux phényles dans lesquels deux atomes de C voisins sont reliés par un groupe méthylènedioxy ou éthylènedioxy

et leurs sels d'addition d'acide.

2. Azidométhyl-2 phényl-5 (1H) dihydro-2,3 benzodiazépines-1,4 de formule générale I selon la revendication 1 dans lesquelles:

le cycle A est non substitué ou substitué par le fluor en position 7, le fluor en position 8, le chlore en position 7, le brome en position 7, un groupe méthoxy en position 7, un groupe méthoxy en position 8, un groupe méthyle en position 7, un groupe trifluorométhyle en position 7, ou un groupe nitro en position 7,

le cycle B est un radical phényle, fluoro-2 phényle, chlore-2 phényle, bromo-2 phényle ou triméthoxy-3,4,5 phényle et

$R_1$ est un atome d'hydrogène, un groupe méthyle ou éthyle.

3. Procédé de préparation d'azidométhyl-2 phényl-5 (1H) dihydro-2,3 benzodiazépines-1,4 de formule générale I

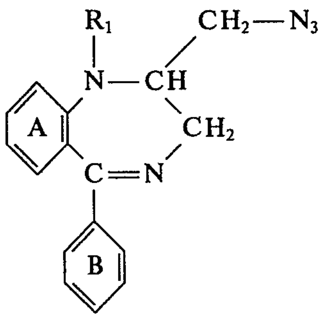

(I)

dans laquelle $R_1$ et les radicaux phényles A et B ont la signification indiquée dans la revendication 1, ainsi que de leurs sels d'addition d'acide, caractérisé en ce que l'on fait réagir des composés de formules générales II et/ou III

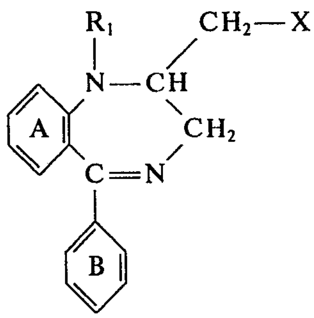

(II)

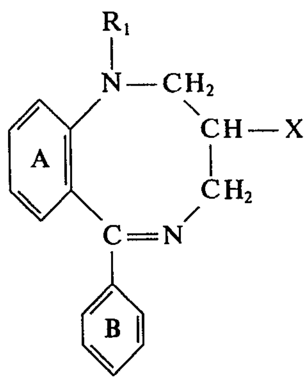

(III)

dans lesquelles X désigne un atome d'halogène et $R_1$, A et B ont la signification ci-dessus, avec un azoture de métal alcalin, dans un solvant inerte à des températures comprises entre −30 et 150°C et que l'on isole les composés de formule générale I sous forme de leur base ou de leurs sels d'addition d'acide.

4. Procédé selon la revendication 3, caractérisé en ce que l'on emploie comme azoture de métal alcalin l'azoture de sodium ou de potassium.

5. Médicaments contenant un ou plusieurs composés selon la revendication 1 et des adjuvants et excipients pharmaceutiques habituels.

**Revendications pour l'etat contractant: AT**

1. Procédé de préparation d'azidométhyl-2 phényl-5 (1H) dihydro-2,3 benzodiazépines-1,4 de formule générale I

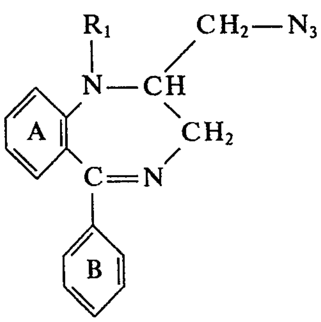

(I)

dans laquelle

$R_1$ est un atome d'hydrogène ou un groupe alkyle à 1 à 4 atomes de carbone,

A et B sont, indépendamment l'un de l'autre, non substitués ou substitués par 1 à 3 substituants choisis parmi un atome d'halogène, un groupe alkylthio à 1 à 4 atomes de carbone alcoxy à 1 à 4 atomes de carbone, alkyle à 1 à 4 atomes de carbone, hydroxy, nitro et trifluorométhyle ou

A et B sont des radicaux phényles dans lesquels deux atomes de C voisins sont reliés par un groupe méthylènedioxy ou éthylènedioxy

et de leurs sels d'addition d'acide, caractérisé en ce que l'on fait réagir des composés de formules générales II et/ou III,

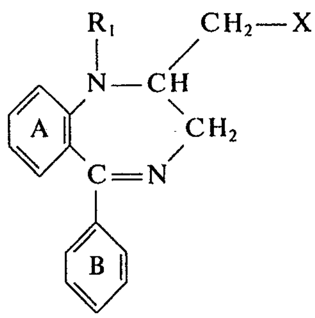
(II)

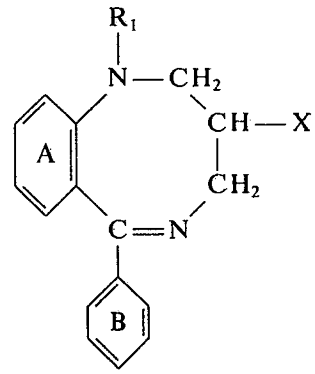
(III)

dans lesquelles X désigne un atome d'halogène et $R_1$, A et B ont la signification ci-dessus, avec un azoture de métal alcalin, dans un solvant inerte à des températures comprises entre −30 et 150°C, et que l'on isole les composés de formule générale I sous forme de leur base ou de leurs sels d'addition d'acide.

2. Procédé selon la revendication 1, caractérisé en ce que l'on prépare des azidométhyl-2 phényl-5 (1H) dihydro-2,3 benzodiazépines-1,4 de formule générale I, dans laquelle

le cycle A est non substitué ou substitué par un atome de fluor en position 7, un atome de fluor en position 8, un atome de chlore en position 7, un atome de brome en position 7, un groupe méthoxy en position 7, un groupe méthoxy en position 8, un groupe méthyle en position 7, un groupe trifluorométhyle en position 7 ou un groupe nitro en position 7,

le cycle B est un radical phényle, fluoro-2 phényle, chlore-2 phényle, bromo-2 phényle ou triméthoxy-3,4,5 phényle et

$R_1$ désigne l'hydrogène, un groupe méthyle ou éthyle.

3. Procédé selon la revendication 1, caractérisé en ce que l'on emploie comme azoture de métal alcalin l'azoture de sodium ou de potassium.